# EUROPÄISCHE PATENTSCHRIFT

(11) **EP 3 566 792 B1**
(45) Veröffentlichungstag und Bekanntmachung des Hinweises auf die Patenterteilung: **22.02.2023**
(21) Anmeldenummer: 19180168.7
(22) Anmeldetag: 15.06.2011
(51) Int. Cl.: B21C 37/02, B21K 23/00, B21H 8/00

(54) **BARREN AUS EDELMETALL UND VERFAHREN ZUR HERSTELLUNG**
BARS MADE OF PRECIOUS METAL AND METHOD OF MANUFACTURING SAME
BARRES EN MÉTAL PRÉCIEUX ET LEUR PROCÉDÉ DE FABRICATION

(30) Priorität: 15.06.2010 DE 102010030128; 20.11.2010 DE 102010044199
(43) Veröffentlichungstag der Anmeldung: 13.11.2019
(62) Teilanmeldung aus: 13174247.0
(73) Patentinhaber: ESG Edelmetall-Service GmbH&Co. KG, 76287 Rheinstetten (DE)
(72) Erfinder: Lochmann, Dominik, 76287 Rheinstetten (DE)
(74) Vertreter: Sommer, Peter

(56) Entgegenhaltungen:
- WO-A1-00/16932
- DE-A1- 2 107 213
- DE-A1- 3 040 052
- DE-T2- 69 415 531
- FR-A- 348 205
- FR-A- 1 152 976
- GB-A- 939 303
- JP-A- 1 133 395
- JP-A- 3 019 393

## Beschreibung

Die Erfindung betrifft einen Barren aus Edelmetall, beispielsweise Gold, Silber, Platin, Palladium oder Legierungen davon und ein Verfahren zur Herstellung eines solchen Barren.

### Stand der Technik

Dosierbare Zahngoldmaterialien sind im Handel als Plättchen erhältlich, wobei die Plättchen durch Herstellung von identischen Abschnitten aus einem gewalzten Goldblech erhalten wurden. Das Goldblech wird durch Auswalzen eines legierten Goldbarrens bereitgestellt, so beschrieben in der DE 10 2004 060 730 A1.

Aus der gattungsgemäßen WO 00/16932 A1 ist ein teilbarer Barren aus Edelmetall mit in einer Reihe hintereinanderliegend angeordneten Abschnitten, die über Stege an einer vorderen und an einer hinteren Seitenfläche eines Abschnitts miteinander verbunden sind, bekannt. Die Stege sind nichtlinear, zum Beispiel Zick-Zack-förmig oder gewunden, sodass jeder Steg Täler an oder nahe der Bodenfläche des Barrens und Spitzen unterhalb der oberen Fläche des Barrens aufweist.

Bei der Herstellung von Goldbarren in kleiner Stückelung, also 1 Gramm, 5 Gramm, 10 Gramm ist es bekannt, auf jedem einzelnen Barren das Gewicht, der Hersteller, die Reinheit des Metalls und das Metall einzuprägen. Barren aus Edelmetall in kleiner Stückelung sind bei Anlegern zwar sehr beliebt, einzeln in der Herstellung aber im Verhältnis zum Metallpreis teuer.

### Darstellung der Erfindung

Der Grundgedanke der Erfindung besteht darin, anstelle vieler einzelner Kleinbarren mit jeweils einer vorgegebenen Masse mk, mehrere solcher Kleinbarren aus einer Tafel oder aus einem Barren mit einer Masse mB auf einmal herzustellen und diese Tafel oder diesen Barren dann zur Ausbildung der Kleinbarren ganz oder teilweise stofflich voneinander zu trennen, wobei die Kleinbarren in einer n x m-Anordnung mit n, m ≥ 2 angeordnet sind. Insgesamt ergibt sich also wegen n x m x mk die Masse mB des Barrens.

Dadurch, dass zunächst ein Barren in Form einer Tafel mit einer Sollmasse mB hergestellt wird, der dann, beispielsweise durch Prägen, in viele Kleinbarren aufgeteilt wird, die im Bedarfsfall ohne Verwendung von Werkzeug von einander abzutrennen bzw. von einem Träger zu lösen sind, lassen sich sowohl die Herstellungskosten verringern als auch die Handhabung der Kleinbarren verbessern.

Die Erfindung betrifft daher einen Barren aus Edelmetall oder einer Edelmetall aufweisenden Legierung mit einer Masse mB., wobei der Barren in viele Kleinbarren mit jeweils einer gewünschten Masse mk unterteilt ist, welche in mehreren Zeilen und mehreren Spalten angeordnet sind. Zwischen den unmittelbar benachbarten Kleinbarren besteht eine stoffliche Verbindung, sodass die Kleinbarren mit ihren unmittelbaren Nachbarn fest verbunden sind. Die stoffliche Verbindung kann beispielsweise in Form einer Brücke oder als Verbindungssteg ausgebildet sein.

Die stoffliche Verbindung weist eine Sollbruchstelle auf. Die stoffliche Verbindung lässt sich ohne Verwendung von Werkzeug trennen. Dabei ist die Biegefestigkeit der Verbindung vorzugsweise so groß, dass ein Verbiegen unter Einwirkung der Schwerkraft vermieden wird und höchstens so groß, dass ein Zerstören der stofflichen Verbindung von Hand durch Biegen oder Brechen möglich ist.

Zusätzlich zu der stofflichen Verbindung kann auf einer Unterseite des Barren ein Trägermaterial angebracht sein.

Die stoffliche Verbindung ist Teil einer in den Barren eingebrachten Vertiefung. Dabei kann eine Vertiefung auf einer Oberseite und eine Vertiefung gegenüberliegend auf einer Unterseite ausgebildet sein und die stoffliche Verbindung kann zu der Oberseite und zu der Unterseite des Barrens beabstandet sein.

Die Vertiefung ist als Rille ausgebildet und die Lage der Rillen im Barren ist so gewählt, dass die durch die Rillen begrenzten Kleinbarren die gewünschte Masse aufweisen.

Vorteilhafterweise kann der Barren in n x m Kleinbarren mit jeweils einer vorgegebenen Masse mk unterteilt sein, wobei n, m jeweils eine natürliche Zahl ≥ 2 ist. Weiter vorteilhafterweise kann m eine natürliche Zahl > 2 sein.

Besonders vorteilhaft ist es, wenn die Vertiefung eingeprägt ist, da hierdurch eine wirtschaftliche Herstellung möglich ist.

Ein weiterer Gegenstand der Erfindung ist ein Verfahren zur Herstellung eines Barren mit einer Masse mB aus Edelmetall oder einer Edelmetall aufweisenden Legierung, bei dem der Barren in einem Herstellungsschritt in viele Kleinbarren mit jeweils einer gewünschten Masse unterteilt wird, welche in mehreren Zeilen und mehreren Spalten angeordnet sind, wobei zwischen den unmittelbar benachbarten Kleinbarren eine stoffliche Verbindung bestehen bleibt.

Die stoffliche Verbindung ist Teil einer Vertiefung. Die Vertiefungen sind in Form von Rillen ausgebildet, wobei die Lage der Rillen in dem Barren so gewählt ist, dass die durch die Rillen begrenzten Kleinbarren die gewünschte Masse aufweisen. Die stoffliche Verbindung weist eine Sollbruchstelle auf und lässt sich ohne Verwendung von Werkzeug trennen.

Vorteilhafterweise erfolgt eine Unterteilung in n x m Kleinbarren mit jeweils einer vorgegebenen Masse mk, wobei n, m jeweils eine natürliche Zahl ≥ 2 ist.

Vorteilhafterweise kann der Barren zur Aufteilung in Kleinbarren geprägt sein.

Bei einem Verfahren zur Herstellung eines Barren mit einer Masse mB aus Edelmetall oder einer Edelmetall aufweisenden Legierung wird ein Edelmetall-Endlosband in einer entsprechenden Dicke gewalzt und einer Umformeinrichtung schrittweise zugeführt und nach einer Umformung wird das Band weitergeschoben. Bei der Umformung wird das Endlosband in eine Reihe aus n x 1 Kleinbarren mit jeweils einer vorgegebenen Masse mk unterteilt, wobei n eine natürliche Zahl ≥ 2 ist und wobei zwischen unmittelbar benachbarten Kleinbarren und dem Endlosband eine stoffliche Verbindung bestehen bleibt. Auf diese Weise entsteht ein Endlosverbundbarren.

Wenn das umgeformte Endlosband zur Herstellung des Barrens nach einer vorgegebenen Zahl von Reihen im Bereich der stofflichen Verbindung zwischen einer Reihe und dem Endlosband getrennt wird, kann der gewünschte Barren entstehen.

Das Endlosband kann eine Breite B größer als die Breite b des herzustellenden Barrens aufweisen, sodass beim Umformen des Endlosbandes zusätzlich zu dem Barren ein überstehender Rand entsteht.

Wenn bei der Umformung ein automatisches Abtrennen des Randes erfolgt, kann nach einer Herstellung einer gewünschte Zahl von Reihen eine Abtrennung vom Endlosband erfolgen und der fertige Barren liegt dann vor.

Vorteilhafterweise kann der beim Umformen entstehende Kleinbarren gleichzeitig beschriftet werden.

### Kurzbeschreibung der Zeichnung

In der Zeichnung sind Ausführungsbeispiele der Erfindung dargestellt. Es zeigt:
- Fig. 1: einen Barren aus Edelmetall in Form einer Tafel mit 4 x 3 Kleinbarren;
- Fig. 2: ein Detail aus Fig. 1;
- Fig. 3: einen Teilschnitt entlang der Linie AA aus Fig. 2;
- Fig. 4A bis 4D: verschiedene Formen einer stofflichen Verbindung zwischen den Kleinbarren einer Tafel;
- Fig. 5A bis 5D: weitere Formen einer stofflichen Verbindung zwischen den Kleinbarren einer Tafel;
- Fig. 6: auf einem Träger angeordnete Kleinbarren ohne stoffliche Verbindungsstelle.
- Fig. 7: ein im Schnitt dargestelltes Endlosband mit Kleinbarren;
- Fig. 8: eine Draufsicht auf das umgeformte Endlosband aus Fig. 7;
- Fig. 9: Kanten eines Werkzeugs für die Herstellung von Kleinbarren.

### Ausführungsbeispiele der Erfindung

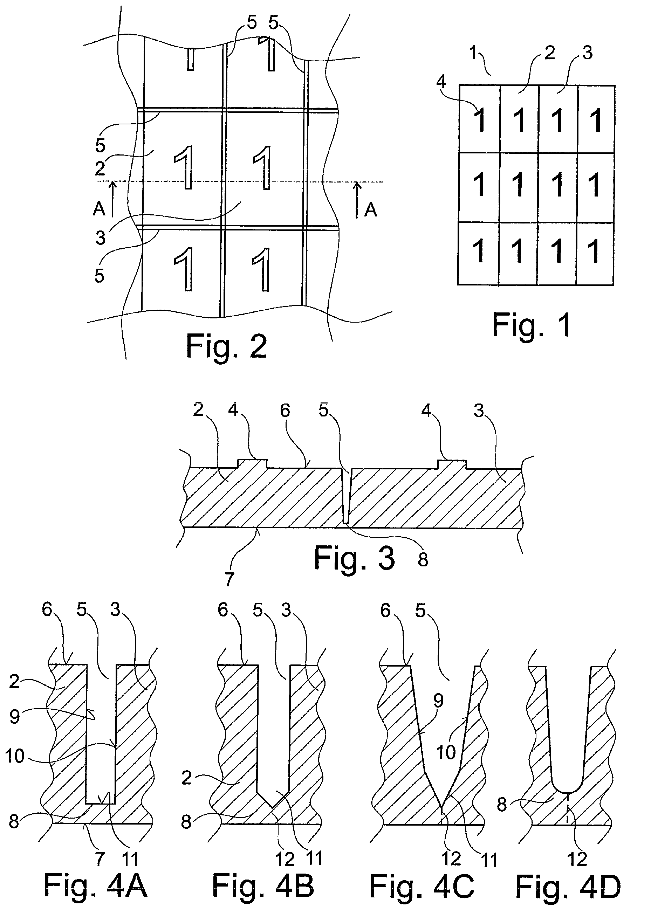

In Fig. 1 ist ein Barren 1 aus Edelmetall in Form einer Tafel mit 4 x 3 Kleinbarren 2,3 gezeigt. Dafür wurde zunächst ein nicht dargestellter Barren in Form einer Tafel mit einer Sollmasse mB hergestellt. Die Bearbeitung des Barrens zur Bereitstellung vieler Kleinbarren 2,3 kann durch Umformen wie beispielsweise Prägen erfolgen, und zwar bereits mit einer Umformung wie einem Prägevorgang, bei dem auch die Angaben 4 über den Kleinbarren 2,3 erzeugt werden, nämlich ein Herstellerlogo, die Masse und die Reinheit, so dass dann die Herstellung einem einzigen Arbeitsgang erfolgen kann. Im Ausführungsbeispiel ist als Angabe 4 die Masse mit 1 angegeben, was 1g bedeutet.

Wie in Fig. 2 im Detail dargestellt, wurden durch den Umformvorgang in den Barren 1 in vorgegebenen Abständen Vertiefungen in Form von Rillen 5 eingebracht, die einen einzelnen Kleinbarren 2 von dem benachbarten Kleinbarren 3 klar erkennbar abgrenzen. Die Lage der Rillen 5 in dem Barren 1 ist so gewählt, dass die durch die Rillen 5 begrenzten Kleinbarren 2,3 die gewünschte Masse aufweisen.

Der Barren kann vor dem Umformvorgang vorzugsweise eine vorgegebene gleichmäßige Dicke aufweisen, damit die Umformung des Barrens mit einem einzigen Prägewerkzeug vorgenommen werden kann und die Masse für die Kleinbarren 2,3 genau genug, also innerhalb der zulässigen Toleranzen, erreicht wird.

Wie ein Barren als Tafel hergestellt werden kann, wird am Beispiel eines Feingoldbarrens mit einer Masse von 100 g erläutert, der in 100 Kleinbarren zu je 1 g unterteilt wird. Dazu wird ein Endlosblechband aus 99,99% Feingold auf eine vorher berechnete Dicke ausgewalzt. Aus diesem Band werden Tafeln von je 100g ausgestanzt.

Diese Tafeln werden in einer für die Prägung von Münzen in ähnlicher Weise bereits bekannten Prägemaschine in einem Arbeitsschritt so geprägt, dass Vertiefungen in Form von Rillen 5 zwischen den einzelnen Kleinbarren entstehen, und dass auf jedem einzelnen 1 g Kleinbarren das Herstellerlogo, das Gewicht und die Reinheit eingeprägt werden.

Die als Rillen ausgebildeten Vertiefungen 5, 5' können so dünn ausgeführt werden, dass das verdrängte Material nur einen vergleichsweise kleinen seitlichen Wulst ergibt, welcher durch den Prägevorgang der Flächen aber sofort wieder abgeflacht werden kann, wenn es gewünscht ist. Dünn bedeutet in diesem Zusammenhang, dass die Breite der Vertiefung geringer als deren Tiefe ist und vorzugsweise höchstens 50% der Tiefe beträgt.

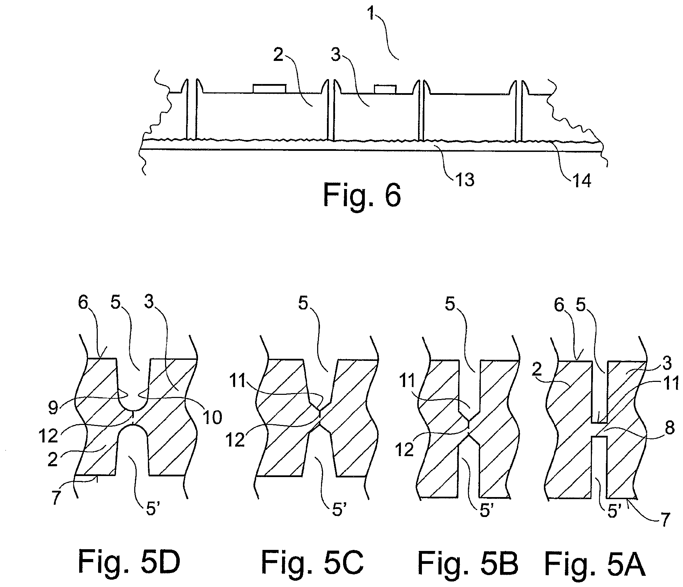

Fig. 3 zeigt einen Teilschnitt entlang der Linie AA aus Fig. 2 und es wird erkennbar, dass die Vertiefungen 5 nicht durch die ganze Tafel hindurch gehen, sondern dass eine von einer Oberseite 6 in Richtung zu einer Unterseite 7 hin ausgehende Rille 5 ausgebildet ist, wobei die stoffliche Verbindung 8 in Form eines Verbindungsstegs, siehe später Fig. 4A-4D, oder einer Brücke, siehe später Fig. 5A-5D besteht.

Die stoffliche Verbindung 8 in der Vertiefung 5 kann dabei in unterschiedlicher Weise ausgebildet sein, wie in den Fig. 4A-4D und 5A-5D beispielhaft gezeigt ist.

In Fig. 4A sind Seitenwände 9,10 der nur von der Oberseite der Tafel her eingebrachten Vertiefung 5 im wesentlichen parallel zueinander und quer zur Oberseite 6 und der Unterseite 7 der Tafel, wobei ein Boden 11 der Vertiefung 5 eben ist und parallel zur Unterseite 7 verläuft. Der Boden 11 ist dabei Teil des Verbindungsstegs der stofflichen Verbindung 8 der benachbarten Kleinbarren 2,3 untereinander.

In Fig. 4B ist im Gegensatz zu Fig. 4A der Boden 11 der Vertiefung zur Unterseite hin zulaufend ausgebildet, sodass in der größten Tiefe des Bodens 11 wegen des geringsten Querschnitts des Verbindungsstegs eine Sollbruchstelle 12 ausgebildet ist.

Mit einer Sollbruchstelle 12 lässt sich die stoffliche Verbindung 8 in der Vertiefung 5 im Bedarfsfall ohne Verwendung von Werkzeug lösen und die Kleinbarren können von einander abgetrennt werden. Dabei wird aufgrund der bekannten Lage der Sollbruchstelle eine planmäßige Verteilung der Massen sichergestellt.

In Fig. 4C sind die Seitenwände der nur von der Oberseite 6 der Tafel her eingebrachten Vertiefung 5 zum Boden hin schräg zulaufend ausgebildet, wobei der Boden 11 der Vertiefung 5 wie in Fig. 4B spitz zulaufend ausgebildet ist. Dadurch ergibt sich eine Sollbruchstelle 12. Im Querschnitt ergibt sich ein Verlauf mit zwei unterschiedlichen Öffnungswinkeln der Vertiefung, wobei der Öffnungswinkel des Bodens 11 größer ist als der Öffnungswinkel der Seitenwände 9, 10.

In Fig. 4D sind die Seitenwände wie in Fig. 4C ausgebildet, wohingegen der Boden 11 gerundet ausgebildet ist, etwa als Rinne. Die Sollbruchstelle 12 liegt auch hier im Bereich des kleinsten Querschnitts des Verbindungsstegs 8.

In den Fig. 5A-5D sind weitere Formen einer stofflichen Verbindung 8 zum Teil mit Sollbruchstelle 12 zwischen den Kleinbarren 2,3 einer Tafel dargestellt, die sich von denen der Fig. 4A-4D dadurch unterscheiden, dass die stoffliche Verbindung in einer sowohl von der Oberseite 6 als auch von der Unterseite 7 eingebrachten Vertiefung 5,5' angeordnet ist. In diesem Fall wird das Material sowohl an die Oberseite 6 als auch an die Unterseite 7 verdrängt und kann, falls gewünscht, im Prägevorgang abgeflacht werden.

In Fig. 6 ist die Tafel 1 mit ihrer Unterseite 7 auf einem Träger 13 angeordnet und mit diesem beispielsweise durch Kleben verbunden, sodass selbst nach einem vollständigen Trennen der Kleinbarren, die dadurch also ohne stoffliche Verbindungsstelle untereinander sind, ein Zusammenhalt gegeben ist. Die stoffliche Trennung kann durch Einbringen einer Vertiefung 5 in Form einer Rille erfolgen, wobei die Vertiefung von der Oberseite 6, also der dem Träger 13 gegenüberliegenden Seite der Tafel ausgeht.

Als Träger 13 kommt beispielsweise eine Trägerplatte in Frage, die mit einer Gummierung 14 ähnlich wie bei Aufklebern beschichtet ist, von der sich die einzelnen Kleinbarren 2,3 leicht ablösen lassen, ohne dass Klebespuren auf den Kleinbarren 2,3 verbleiben. Die Trägerplatte kann entweder aus dickerem Papier, einem Karton oder Kunststoff bestehen.

Die Kleinbarren 2,3 lassen sich dann einzeln oder zu mehreren von dem Träger lösen, die nicht gelösten übrigen Kleinbarren bleiben auf dem Träger 13 und sind wieder gemeinsam handhabbar.

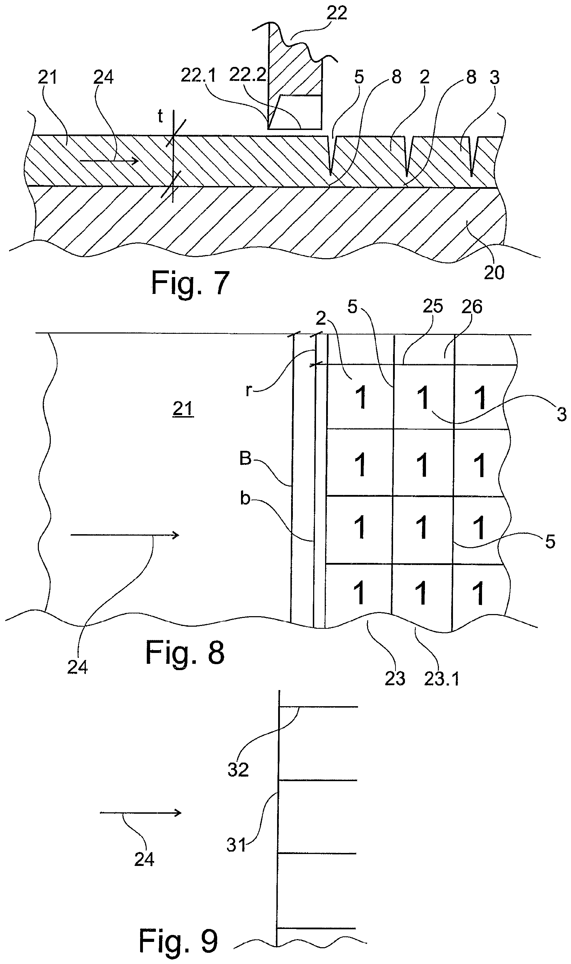

In einer weiteren, in den Fig. 7-9 dargestellten Ausführungsform wird der Barren 1 so hergestellt, dass ausgehend von einem in Fig. 7 im Schnitt dargestellten, auf eine vorberechnete Dicke t ausgewalztes Endlosband 21 aus 99,99% Feingold eine schrittweise Umformung des Endlosbandes 21 unter Ausbildung der Kleinbarren erfolgt. Dabei wird das auf einem Tisch 20 aufliegende Endlosblechband 21 an eine Umformstation 22 herangeführt und in einem Umformschritt werden Kleinbarren 2 erzeugt, die mit einem im vorherigen Umformschritt erzeugten Kleinbarren 3 und zusätzlich auch noch mit dem noch nicht umgeformten Endlosband 21 jeweils am Boden einer als Rille ausgebildete Vertiefung 5 eine stoffliche Verbindung 8 aufweisen. Die als Rille ausgebildete Vertiefung 5 wird mit einer scharfen Kante 22.1 erzeugt, eine weitere scharfe Kante 22.2 ist für nicht dargestellte Rillen in Zufuhrrichtung 24 gezeigt.

Wie aus der Draufsicht aus Fig. 8 ersichtlich ist, sind die Kleinbarren 2, 3 in jeweils einer Reihe 23, 23.1. quer zu der Zufuhrrichtung 24 des Endlosbandes 21 angeordnet und durch die als Rille ausgebildete Vertiefung 5 voneinander abgesetzt.

Ist die Umformung einer Reihe 23 abgeschlossen, wird das Endlosblechband 21 gegenüber der nicht dargestellten Umformeinrichtung einen Schritt weiter vorgeschoben und es findet eine erneute Umformung statt, um die nächste Reihe von Kleinbarren 2, 3 zu erzeugen. Der Barren selbst kann dann durch geeignetes Abtrennen auf die gewünschte Anzahl von Reihen 23 von Kleinbarren 2, 3 gebracht werden.

Wenn das Endlosblechband 21 eine Breite B größer als die Breite b des herzustellenden Barren aufweist, wird jeder Kleinbarren 2, 3 bei seiner Herstellung denselben Umformungen unterworfen. Allerdings entsteht durch das seitliche Übermaß des Endlosblechbandes 21 gegenüber dem Barren 1 dann ein durch eine als Rille ausgebildete Vertiefung 25 abgesetzter aber gleichwohl noch stofflich verbundener seitlicher Rand 26 der Breite r, der sich in Zufuhrrichtung 25 erstreckt. Der Rand 26 kann bereits beim Umformen abgetrennt werden oder er wird erst nach dem Umformen abgetrennt.

Auch bei dieser Umformung kann die Erzeugung von Vertiefungen 5 sowohl von der Oberseite her als auch von der Unterseite her des Endlosbandes 21 erfolgen. Die zusätzliche Umformung von der Unterseite her ist grundsätzlich dann vorteilhaft, wenn die Dicke des Barrens so groß ist, dass die Umformung von nur einer Seite her nicht ausreicht.

Die durch die Einbringung von Vertiefungen 5 in den Barren erzeugten Sollbruchstellen im Bereich der stofflichen Verbindung 8 können einen Öffnungswinkel von etwa 10° bis 60° aufweisen und die stoffliche Verbindung 8 kann eine Dicke von 0,05mm bis 0,4mm aufweisen, wobei auch andere Dicken noch ein Abtrennen von Hand ermöglichen.

In Fig. 9 sind Kanten 31, 32 eines Werkzeugs für die Herstellung von Kleinbarren dargestellt. Eine Kante 31 verläuft quer zur Zuführrichtung 24 und erzeugt die als Rille ausgebildete Vertiefung 5, eine weitere Kante 32 in Zuführrichtung 24 erzeugt beispielsweise die als Rille ausgebildete Vertiefung 25 am Rand 26 eines Kleinbarrens 2, 3 aus Fig. 8. Die Kanten 31, 32 werden in das Endlosband gedrückt und verdrängen das Material, sodass eine Vertiefung entsteht, wobei gleichzeitig eine stoffliche Verbindung bestehen bleibt, was hier nicht dargestellt ist.

Auch bei der Ausführungsform nach den Fig. 7-9 ist es möglich, anstelle einer stofflichen Verbindung ein Träger vorzusehen, der mit dem Endlosband verbunden ist.

Die Barren werden beispielsweise in den nachfolgend angegebenen Größen hergestellt. Für Gold 100x1g: 74mm x 105mm x 0,667mm oder 85mm x 150mm x 0,406mm; für Silber: 100x1g: 74mm x 105mm x 1,226mm; für Platin: 100x1g: 74mm x 105mm x 0,602mm und für Palladium: 100x1g : 74mm x 105mm x 1,073mm.

Es lassen sich hier also zwei Herstellungsvarianten unterscheiden, wobei andere Herstellungsverfahren, etwa durch Gießen, nicht ausgeschlossen werden. In der ersten Variante wird ein Edelmetallblech auf die fertigen Endmaße geschnitten. Das zugeschnittene Blech kommt dann in eine normale Prägemaschine, wie sie auch für Münzen oder normale Edelmetallbarren eingesetzt wird und wird dort mit entsprechend ausgeformten Prägestempeln unter hohem Druck in die Endform geprägt.

In der zweiten Variante wird ein Edelmetall-Endlosband in entsprechender Dicke gewalzt und in einer Stanzmaschine jeweils eine komplette Reihe, etwa 10 Kleinbarren zu 1g gleichzeitig gekerbt und beschriftet. Danach wird das Band weitergeschoben, so dass ein Endlosverbund entsteht, der dann jeweils nach 10 Reihen gekappt werden kann, um einen Barrenverbund in einer Stückelung von 10x10x1g zu erhalten.

Mit beiden Varianten können auch hochglänzende Oberfläche hergestellt werden.

## Patentansprüche

1. Barren aus Edelmetall oder einer Edelmetall aufweisenden Legierung, mit einer Masse mB, **dadurch gekennzeichnet, dass** der Barren (1) in viele Kleinbarren (2,3) mit jeweils einer gewünschten Masse mk unterteilt ist, welche in mehreren Zeilen und mehreren Spalten angeordnet sind und dass zwischen den unmittelbar benachbarten Kleinbarren (2,3) eine stoffliche Verbindung (8) besteht, dass die stoffliche Verbindung (8) Teil von in den Barren (1) eingebrachten Vertiefungen (5,5') ist, dass die Vertiefungen in Form von Rillen ausgebildet sind und dass die Lage der Rillen in dem Barren so gewählt ist, dass die durch die Rillen begrenzten Kleinbarren (2, 3) die gewünschte Masse aufweisen und dass die stoffliche Verbindung eine Sollbruchstelle (12) aufweist und sich ohne Verwendung von Werkzeug trennen lässt.

2. Barren nach Anspruch 1, **dadurch gekennzeichnet, dass** der Barren in n x m Kleinbarren mit jeweils einer Masse mk unterteilt ist, wobei n, m jeweils eine natürliche Zahl ≥ 2 ist.

3. Barren nach Anspruch 2, **dadurch gekennzeichnet, dass** m eine natürliche Zahl > 2 ist.

4. Barren nach einem der Ansprüche 1 bis 3, **dadurch gekennzeichnet, dass** auf einer Unterseite (7) des Barrens ein Träger (13) angebracht ist.

5. Barren nach einem der Ansprüche 1 bis 4, **dadurch gekennzeichnet, dass** eine Vertiefung (5) auf einer Oberseite (6) und eine Vertiefung (5') gegenüberliegend auf einer Unterseite (7) ausgebildet ist und dass die stoffliche Verbindung (8) zu der Oberseite (6) und zu der Unterseite (7) des Barrens beabstandet ist.

6. Barren nach einem der Ansprüche 1 bis 5, **dadurch gekennzeichnet, dass** die Vertiefung (5, 5') zulaufend ausgebildet ist und dass die stoffliche Verbindung (8) in der größten Tiefe der Vertiefung die Sollbruchstelle (12) aufweist.

7. Barren nach einem der Ansprüche 1 bis 6, **dadurch gekennzeichnet, dass** der Barren als die fertigen Endmaße aufweisende Tafel mit einer Masse mB hergestellt ist und dass in diese Tafel die Vertiefungen eingeprägt sind.

8. Barren nach einem der Ansprüche 1 bis 7, **dadurch gekennzeichnet, dass** die stoffliche Verbindung (8) Teil einer in den Barren (1) eingebrachten, zulaufend ausgebildeten Vertiefung (5, 5') ist und dass die stoffliche Verbindung (8) in der größten Tiefe der Vertiefung eine Sollbruchstelle (12) aufweist und eine Dicke aufweist, die ein Abtrennen von Hand ermöglicht.

9. Barren nach Anspruch 8, **dadurch gekennzeichnet, dass** die Sollbruchstelle (12) im Bereich der stofflichen Verbindung (8) einen Öffnungswinkel von 10° bis 60° aufweist.

10. Barren nach einem der Ansprüche 1 bis 9, **dadurch gekennzeichnet, dass** die stoffliche Verbindung (8) eine Dicke von 0,05 mm bis 0,4 mm aufweist.

11. Barren nach einem der Ansprüche 8 bis 10, **dadurch gekennzeichnet, dass** die Biegefestigkeit der stofflichen Verbindung so groß ist, dass ein Verbiegen unter Einwirkung der Schwerkraft vermieden wird und höchstens so groß, dass ein Zerstören der stofflichen Verbindung von Hand durch Biegen oder Brechen möglich ist.

12. Barren nach einem der Ansprüche 1 bis 11, **dadurch gekennzeichnet, dass** eine Vertiefung (5) auf einer Oberseite (6) und eine Vertiefung (5') gegenüberliegend auf einer Unterseite (7) ausgebildet ist und dass die stoffliche Verbindung (8) zu der Oberseite (6) und zu der Unterseite (7) des Barrens beabstandet ist.

13. Verfahren zur Herstellung eines Barren mit einer Masse mB aus Edelmetall oder einer Edelmetall aufweisenden Legierung, **dadurch gekennzeichnet, dass** der Barren (1) in einem Herstellungsschritt in viele Kleinbarren (2, 3) mit jeweils einer gewünschten Masse mk unterteilt wird, welche in mehreren Zeilen und mehreren Spalten angeordnet sind, wobei zwischen den unmittelbar benachbarten Kleinbarren (2, 3) eine stoffliche Verbindung (8) als Teil von in den Barren (1) eingebrachten Vertiefungen bestehen bleibt, wobei die Vertiefungen in Form von Rillen ausgebildet und wobei die Lage der Rillen in dem Barren so gewählt ist, dass die durch die Rillen begrenzten Kleinbarren (2, 3) die gewünschte Masse aufweisen und dass die stoffliche Verbindung (8) eine Sollbruchstelle aufweist und sich ohne Verwendung von Werkzeug trennen lässt.

14. Verfahren nach Anspruch 13, **dadurch gekennzeichnet, dass** eine Unterteilung in n x m Kleinbarren mit jeweils einer vorgegebenen Masse mk erfolgt, wobei n, m jeweils eine natürliche Zahl ≥ 2 ist.

15. Verfahren nach Anspruch 14, **dadurch gekennzeichnet, dass** m eine natürliche Zahl > 2 ist.

16. Verfahren nach einem der Ansprüche 13 bis 15, **dadurch gekennzeichnet, dass** der Kleinbarren bei seiner Herstellung gleichzeitig beschriftet wird.

17. Verfahren nach einem der Ansprüche 13 bis 16, **dadurch gekennzeichnet, dass** die durch Einbringung von Vertiefungen erzeugten Sollbruchstellen im Bereich der stofflichen Verbindung (8) mit einem Öffnungswinkel von 10° bis 60° ausgebildet werden.

18. Verfahren nach einem der Ansprüche 13 bis 17, **dadurch gekennzeichnet, dass** die stoffliche Verbindung (8) mit einer Dicke von 0,05 mm bis 0,4 mm hergestellt wird.

## Claims

1. Bar of noble metal or an alloy containing noble metal, having a mass mB, **characterised in that** the bar (1) is subdivided into many miniature bars (2, 3) each having a desired mass mk, which are arranged in a plurality of rows and a plurality of columns and **in that** between the directly adjacent miniature bars (2, 3) there exists an interconnection of solid material (8), **in that** the interconnection of solid material (8) is a part of depressions (5, 5') formed in the bar (1), **in that** the depressions are in the form of grooves and **in that** the position of the grooves in the bar is selected such that the miniature bars (2, 3) delimited by the grooves have the desired mass and **in that** the interconnection of solid material has a break point (12) and can be separated without the use of a tool.

2. Bar according to claim 1, **characterised in that** the bar is subdivided into n x m miniature bars each having a mass mk, wherein n, m is in each case a natural number ≥ 2.

3. Bar according to claim 2, **characterised in that** m is a natural number > 2.

4. Bar according to any one of claims 1 to 3, **characterised in that** a backing (13) is applied to an underside (7) of the bar.

5. Bar according to any one of claims 1 to 4, **characterised in that** a depression (5) is formed on the top surface (6) and a depression (5') is formed oppositely thereto on the underside (7) and **in that** the interconnection of solid material (8) is set at a distance from the top surface (6) and from the underside (7) of the bar.

6. Bar according to any one of claims 1 to 5, **characterised in that** the depression (5, 5') is formed so as to be tapered and **in that** the interconnection of solid material (8) has the break point (12) at the lowest depth of the depression.

7. Bar according to any one of claims 1 to 6, **characterised in that** the bar is manufactured as a tablet having the desired size and a mass mB, and **in that** the depressions are impressed into said tablet.

8. Bar according to any one of claims 1 to 7, **characterised in that** the interconnection of solid material (8) is part of a tapered depression (5, 5') formed in the bar (1), and **in that** the interconnection of solid material (8) has a predetermined break point (12) in the deepest region of the depression and has a thickness enabling manual break-off.

9. Bar according to claim 8, **characterised in that** the break point (12) in the region of the material connection (8) has an aperture angle of 10° to 60°.

10. Bar according to any one of claims 1 to 9, **characterised in that** the interconnection of solid material (8) has a thickness of 0.05 mm to 0.4 mm.

11. Bar according to any one of claims 8 to 10, **characterised in that** the flexural strength of the interconnection of solid material is sufficiently great that bending under the influence of gravity is avoided and does not exceed a strength at which manual destruction of the interconnection of solid material by means of bending or breaking is possible.

12. Bar according to any one of claims 1 to 11, **characterised in that** a depression (5) is formed on the top surface (6) and a depression (5') is formed oppositely on the underside (7) and **in that** the interconnection of solid material (8) is set at a distance from the top surface (6) and from the underside (7) of the bar.

13. Method for the production of a bar, having a mass mB, consisting of noble metal or an alloy containing a noble metal, **characterised in that,** in one manufactuing step, the bar (1) is subdivided into many miniature bars (2, 3) each having a desired mass mk, which are arranged in a plurality of rows and a plurality of columns, wherein between the directly adjacent miniature bars (2, 3) an interconnection of solid material (8) remains as a part of depressions formed in the bar (1), wherein the depressions have the form of grooves and wherein the position of the grooves in the bar is selected such that the miniature bars (2, 3) delimited by the grooves have the desired mass and **in that** the interconnection of solid material (8) has a break point and can be separated without the use of a tool.

14. Method according to claim 13, **characterised in that** a subdivision into n x m miniature bars each with a predetermined mass mk takes place, wherein n, m is in each case a natural number ≥ 2.

15. Method according to claim 14, **characterised in that** m is a natural number > 2.

16. Method according to any one of claims 13 to 15, **characterised in that** the miniature bar is inscribed during production thereof.

17. Method according to any one of claims 13 to 16, **characterised in that** break points created by the introduction of depressions are formed in the region of the interconnection of solid material (8) with an aperture angle from 10° to 60°.

18. Method according to any one of claims 13 to 17, **characterised in that** the interconnection of solid material (8) is produced with a thickness of 0.05 mm to 0.4 mm.

## Revendications

1. Lingot en métal précieux ou un alliage présentant un métal précieux, avec une masse mB, **caractérisé en ce que** le lingot (1) est divisé en de nombreux petits lingots (2, 3) avec respectivement une masse souhaitée mk qui sont agencés en plusieurs lignes et plusieurs colonnes et qu'entre les petits lingots (2, 3) directement contigus une liaison de matière (8) existe, que la liaison de matière (8) fait partie de cavités (5, 5') pratiquées dans les lingots (1), que les cavités sont réalisées sous la forme de cannelures et que la position des cannelures dans le lingot est choisie de sorte que les petits lingots (2, 3) délimités par les cannelures présentent la masse souhaitée et que la liaison de matière présente une zone de rupture théorique (12) et peut être séparée sans utilisation d'outil.

2. Lingot selon la revendication 1, **caractérisé en ce que** le lingot est divisé en n x m petits lingots avec respectivement une masse mk, dans lequel n, m est respectivement un entier naturel ≥ 2.

3. Lingot selon la revendication 2, **caractérisé en ce que** m est un entier naturel > 2.

4. Lingot selon l'une quelconque des revendications 1 à 3, **caractérisé en ce qu'**un support (13) est monté sur un côté inférieur (7) du lingot.

5. Lingot selon l'une quelconque des revendications 1 à 4, **caractérisé en ce qu'**une cavité (5) est réalisée sur un côté supérieur (6) et une cavité (5') à l'opposé sur un côté inférieur (7) et que la liaison de matière (8) est espacée du côté supérieur (6) et du côté inférieur (7) du lingot.

6. Lingot selon l'une quelconque des revendications 1 à 5, **caractérisé en ce que** la cavité (5, 5') est réalisée de manière effilée et que la liaison de matière (8) présente dans la plus grande profondeur de la cavité la zone de rupture théorique (12).

7. Lingot selon l'une quelconque des revendications 1 à 6, **caractérisé en ce que** le lingot est fabriqué comme tablette présentant les cotes finies avec une masse mB et que dans cette tablette les cavités sont gaufrées.

8. Lingot selon l'une quelconque des revendications 1 à 7, **caractérisé en ce que** la liaison de matière (8) fait partie d'une cavité (5, 5') réalisée de manière effilée, pratiquée dans le lingot (1) et que la liaison de matière (8) présente dans la plus grande profondeur de la cavité une zone de rupture théorique (12) et présente une épaisseur qui permet une séparation manuelle.

9. Lingot selon la revendication 8, **caractérisé en ce que** la zone de rupture théorique (12) présente dans la zone de la liaison de matière (8) un angle d'ouverture de 10° à 60°.

10. Lingot selon l'une quelconque des revendications 1 à 9, **caractérisé en ce que** la liaison de matière (8) présente une épaisseur de 0,05 mm à 0,4 mm.

11. Lingot selon l'une quelconque des revendications 8 à 10, **caractérisé en ce que** la résistance à la flexion de la liaison de matière est si grande qu'une flexion sous l'action de la force de gravité est évitée et au plus si grande qu'une destruction de la liaison de matière de la main est possible par flexion ou rupture.

12. Lingot selon l'une quelconque des revendications 1 à 11, **caractérisé en ce qu'**une cavité (5) est réalisée sur un côté supérieur (6) et une cavité (5') à l'opposé sur un côté inférieur (7) et que la liaison de matière (8) est espacée du côté supérieur (6) et du côté inférieur (7) du lingot.

13. Procédé de fabrication d'un lingot avec une masse mB en métal précieux ou un alliage présentant un métal précieux, **caractérisé en ce que** le lingot (1) est divisé dans une étape de fabrication en de nombreux petits lingots (2, 3) avec respectivement une masse souhaitée mK, qui sont agencés en plusieurs lignes et plusieurs colonnes, dans lequel entre les petits lingots (2, 3) directement contigus une liaison de matière (8) reste comme partie de cavités pratiquées dans les lingots (1), dans lequel les cavités sont réalisées en forme de cannelures et dans lequel la position des cannelures dans le lingot est choisie de sorte que les petits lingots (2, 3) délimités par les cannelures présentent la masse souhaitée et que la liaison de matière (8) présente une zone de rupture théorique et peut être séparée sans utilisation d'outil.

14. Procédé selon la revendication 13, **caractérisé en ce qu'**une division en n x m petits lingots est effectuée avec respectivement une masse prédéfinie mK, dans lequel n, m est respectivement un entier naturel ≥ 2.

15. Procédé selon la revendication 14, **caractérisé en ce que** m est un entier naturel > 2.

16. Procédé selon l'une quelconque des revendications 13 à 15, **caractérisé en ce que** le petit lingot reçoit une inscription simultanément lors de sa fabrication.

17. Procédé selon l'une quelconque des revendications 13 à 16, **caractérisé en ce que** les zones de rupture théorique par réalisation de cavités sont réalisées dans la zone de liaison de matière (8) avec un angle d'ouverture de 10° à 60°.

18. Procédé selon l'une quelconque des revendications 13 à 17, **caractérisé en ce que** la liaison de matière (8) est établie avec une épaisseur de 0,05 mm à 0,4 mm.
